Europäisches Patentamt

European Patent Office

Office européen des brevets

(19)

(11) Numéro de publication: **0 246 152 B1**

(12) **FASCICULE DE BREVET EUROPEEN**

(45) Date de publication de fascicule du brevet:
21.08.91

(51) Int. Cl.⁵: **G01N 33/78,** G01N 33/543,
//G01N33/535

(21) Numéro de dépôt: **87401061.4**

(22) Date de dépôt: **11.05.87**

(54) **Procédé de dosage immunologique des hormones thyroidiennes T3 et/ou T4 utilisant la thyroglobuline.**

(30) Priorité: **12.05.86 FR 8606763**

(43) Date de publication de la demande:
**19.11.87 Bulletin 87/47**

(45) Mention de la délivrance du brevet:
**21.08.91 Bulletin 91/34**

(84) États contractants désignés:
**AT BE CH DE ES FR GB GR IT LI LU NL SE**

(56) Documents cités:
**EP-A- 0 089 806**
**EP-A- 0 165 669**
**US-A- 4 481 298**

**CLINICAL CHEMISTRY, vol.28, no.7, 1982;**
**C.BLAKE et al., pp.1469-1470**

**CHEMICAL ABSTRACTS, vol.94, no.9, 02 mars 1981, Columbus, OH (US); P.-L.TAO et al., p.323, no.61029d**

**CLINICAL CHEMISTRY, vol. 28, no. 4, 1982; H.H.WEETALL et al., pp.666-671**

(73) Titulaire: **CIS BIO INTERNATIONAL**
**RN 306**
**F-91000 Saclay(FR)**

(72) Inventeur: **Ferrua, Bernard**
**25 rue de Roquebillière**
**F-06300 Nice(FR)**
Inventeur: **Moulin, Claude**
**5 Domaine de Paniscoule**
**F-30200 Bagnols sur Ceze(FR)**

(74) Mandataire: **Pottier, Pierre Société BREVATO-ME et al**
**25, Rue de Ponthieu**
**F-75008 Paris(FR)**

Rank Xerox (UK) Business Services

## Description

La présente invention a pour objet un procédé de dosage immunologique des hormones thyroïdiennes $T_3$ et/ou $T_4$.

La glande thyroïde est une glande endocrine située à la base du cou qui synthétise deux hormones peptidiques, la triiodothyronine $T_3$ et la thyroxine $T_4$ par condensation de deux molécules de thyrosine iodée qui sont la monoiodothyrosine et la diiodothyrosine.

Cette synthèse a lieu à partir d'une protéine thyroïdienne, la thyroglobuline qui est également la forme de réserve des hormones thyroïdiennes. La libération de ces hormones dans la circulation sanguine se fait par un processus protéolytique et ces hormones sont transportées par trois protéines sériques : le TBG (thyroxine binding globuline), la TBPA (thyroxine binding prealbumin) et l'albumine. Les formes libres et liées des hormones coexistent toutefois dans le sang dans un état d'équilibre dynamique régi par la loi d'action de masse.

Les hormones thyroïdiennes exercent leur activité sur la majorité des tissus à l'exception du cerveau ; ainsi, elles stimulent la consommation en oxygène de la plupart des cellules, participent à la régulation du métabolisme des lipides et des glucides et sont nécessaires à l'évolution d'une croissance normale. Cependant, il est généralement admis que seules les formes libres de l'hormone sont biologiquement actives et peuvent pénétrer dans les cellules pour exercer leur action physiologique.

Aussi, des procédés permettant de doser non seulement la concentration totale des hormones $T_3$ et $T_4$ mais aussi la concentration de ces hormones sous forme libre sont d'un grand intérêt pour le diagnostic de l'hyperthyroïdie ou de l'hypothyroïdie car la détermination du taux de $T_4$ et/ou de $T_3$ total peut conduire à des erreurs de diagnostic d'hyperthyroïdie ou d'hypothyroïdie provoquée par une altération des protéines de transport. En effet, la concentration des protéines de liaison peut augmenter par exemple lors de la grossesse, lors de maladies hépatiques et lors de prises d'oestrogènes ou d'opiacés, et le taux de $T_4$ ou de $T_3$ total augmente alors de ce fait de façon concomitante, ce qui peut conduire à un diagnostic d'hyperthyroïdie alors que les taux de $T_3$ ou $T_4$ libre sont normaux et que le patient présente un état euthyroïdien.

Inversement, le taux de $T_3$ ou $T_4$ total peut être abaissé par la diminution du taux des protéines liantes, ce qui pourrait conduire à un diagnostic d'hypothyroïdie alors que le taux de $T_3$ ou de $T_4$ libre est normal.

Aussi, il est préférable de pouvoir déterminer les taux de $T_3$ ou de $T_4$ libres pour mieux apprécier l'état du patient, ceux-ci pouvant être déterminés soit directement, soit à partir des concentrations totales en $T_3$ et $T_4$ et de l'indice de saturation des protéines porteuses.

Les faibles taux des hormones thyroïdiennes $T_3$ et $T_4$ rencontrés dans les sérums humains ou animaux nécessitent l'emploi de méthodes de dosages sensibles.

Les méthodes couramment utilisées pour des dosages de ce type font appel aux techniques immunologiques. Parmi celles-ci, on a utilisé tout d'abord les dosages radioimmunologiques utilisant de la $T_4$ ou $T_3$ marquée à l'iode 125. Plus récemment, on a développé des techniques de dosage enzymoimmunologique, soit en phase hétérogène, soit en phase homogène.

Parmi les méthodes en phase homogène, on connaît des dosages basés sur la polarisation de fluorescence, sur le transfert de fluorescence ou sur une inhibition de l'enzyme. Ces dosages ont l'avantage de pouvoir être complètement automatisés, mais ils nécessitent l'utilisation d'appareillages sophistiqués.

Parmi les méthodes en phase hétérogène, on connaît un procédé de dosage de la $T_3$ ou la $T_4$ libre ou totale qui consiste à mettre en compétition $T_3$ ou $T_4$ et une quantité connue de $T_3$ ou $T_4$ marquée par une enzyme pour un nombre limité de sites anticorps spécifiques de l'hormone (H.V. Weetall et al Clin. Chem. Vol.28, n°4 (1982), p.666-671; SCHALL et al., Clin. Chem., 1978, 24, 1801-1804 ; et ALBERT et al., Ed.S.B. Pal, 1978, p. 153-174). Pour réaliser le même type de dosage en phase hétérogène, on pourrait aussi envisager de mettre en compétition l'hormone à doser et une quantité déterminée d'hormone fixée sur un support solide pour une quantité limitée de sites actifs d'un anticorps marqué spécifique de l'hormone. (Masao Ito et al., Clin. Chem. 30/10, 1682-1685 (1984) et Gnemmi et al., Enzyme Labelled Immunoassay of Hormone and Drugs, 1978, Walter de Gruyter et Co. Berlin-New-York).

Cependant, ces deux modes de dosage en phase hétérogène sont difficiles à mettre en oeuvre avec les hormones $T_3$ et $T_4$, car celles-ci perdent généralement leur activité antigénique lorsqu'on les fixe sur un support solide ou leur activité biologique et/ou antigénique lorsqu'on les couple à une macromolécule. De plus, lorsque l'on veut utiliser cette méthode pour le dosage de la $T_3$ et/ou de la $T_4$ sous forme libre, il est nécessaire que les molécules de $T_3$ et/ou de $T_4$ marquées ne soient pas reconnues par les protéines de transport des hormones $T_3$ et $T_4$.

En revanche, on pourrait réaliser le dosage en couplant chimiquement l'hormone $T_3$ ou $T_4$ à une poudre mais dans ce cas la réalisation du dosage pose des problèmes de lavage.

La présente invention a précisément pour objet un procédé de dosage immunologique des hormones thyroïdiennes $T_3$ et/ou $T_4$ qui permet de résoudre les problèmes évoqués ci-dessus.

Selon l'invention, le procédé de dosage immunologique de la thyroxine $T_4$ et/ou de la triiodothyronine $T_3$ présentes sous forme libre, c'est-à-dire non liées aux protéines vectrices, dans un milieu réactionnel, se caractérise en ce qu'il consiste à mettre en compétition $T_3$ et/ou $T_4$ à doser avec de la thyroglobuline pour les sites d'anticorps anti-$T_3$ et/ou anti-$T_4$ présents en quantité limitée, et à déterminer ensuite soit la quantité de thyroglobuline fixée aux anticorps anti-$T_3$ et/ou anti-$T_4$, soit la quantité de thyroglobuline non fixée aux anticorps anti-$T_3$ et/ou anti-$T_4$.

On précise que dans la suite du texte, les termes "sous forme libre" appliqués à $T_3$ et $T_4$ signifient qu'il s'agit de $T_3$ et $T_4$ non liées aux protéines vectrices. Ceci est aussi bien valable pour le dosage de la $T_3$ ou $T_4$ libre que pour le dosage de la $T_3$ ou $T_4$ totale tel que connu dans la littérature.

Le procédé de l'invention consiste ainsi à utiliser la thyroglobuline comme compétiteur pour la réaction de compétition vis-à-vis des sites anticorps. Ceci permet d'obtenir de nombreux avantages car la thyroglobuline peut être fixée sur une phase solide ou être couplée à une molécule ou un atome marqueur, tout en étant toujours reconnue par des anticorps anti-$T_3$ et anti-$T_4$. Par ailleurs, le dosage n'est pas perturbé par les protéines de transport des hormones $T_3$ et $T_4$, puisque l'on utilise comme compétiteur la thyroglobuline au lieu d'hormones $T_3$ et/ou $T_4$ marquées ou des anticorps marqués.

La thryroglobuline est une glycoprotéine de structure complexe qui est le produit de départ de la synthèse des hormones $T_3$ et $T_4$ et contient de ce fait les séquences de ces hormones. Cependant, on ne pouvait supposer que dans cette structure complexe, ces séquences soient reconnues par des anticorps spcécifiques des hormones $T_3$ et $T_4$. En effet, les études portant sur la structure de la thyroglobuline comme celle publiée par C. Marriq et al. dans Eur. J. Biochem. 111, 33-47 (1980) ont simplement montré qu'il existait dans cette structure plusieurs sites de formation des hormones $T_3$ et $T_4$ et que la biosynthèse de l'hormone $T_3$ ne pouvait sans doute se produire que dans une zone bien délimitée de la molécule, tandis que la biosynthèse de l'hormone $T_4$ pouvait prendre place sur un nombre plus important de sites. Les résultats de cette étude démontraient que la thyroglobuline ne présentait pas de réactivité vis-à-vis des anticorps anti-$T_3$ ou anti-$T_4$.

Dans le procédé de l'invention, on peut utiliser de la thyroglobuline provenant de différentes espèces animales, par exemple de la thyroglobuline de boeuf ou de la thyroglobuline de porc.

On peut utiliser par ailleurs de la thyroglobuline sous différentes formes, par exemple sous forme monomère, sous forme native, sous forme dénaturée, sous forme polymérisée ou hydrolysée.

Le procédé de l'invention peut être utilisé pour déterminer, soit la concentration totale en $T_4$ ou $T_3$ d'un échantillon biologique, soit la concentration en $T_4$ ou $T_3$ libre de cet échantillon. En effet, comme on l'a vu ci-dessus, le procédé de dosage de l'invention permet de doser la $T_3$ et/ou la $T_4$ libre. Cependant, dans le cas où l'on veut déterminer la concentration totale en $T_4$ ou $T_3$ d'un échantillon, on soumet celui-ci à un traitement pour libérer les hormones $T_3$ et $T_4$ liées à des protéines porteuses de l'échantillon. Ce traitement peut consister en l'addition d'un inhibiteur de la liaison de $T_3$ et/ou $T_4$ avec leurs protéines vectrices, cet inhibiteur étant par exemple l'acide salicylique, du merthiolate ou l'acide 8-anilino-1-naphtalène sulfonique (ANS). A l'aide de ce traitement l'échantillon peut être utilisé directement dans le procédé de dosage de l'invention.

Aussi dans la suite du présent texte, on entendra par dosage de $T_3$ et/ou $T_4$, aussi bien le dosage de la concentration totale en $T_3$ et/ou $T_4$ que le dosage de la concentration en $T_3$ et/ou $T_4$ libre.

Pour mettre en oeuvre le procédé de l'invention, on effectue tout d'abord une réaction de compétition entre la $T_3$ et/ou la $T_4$ à doser et la thyroglobuline pour les sites des anticorps anti-$T_3$ et/ou anti-$T_4$ présents en quantité limitée. A la fin de cette réaction, on obtient donc de la thyroglobuline liée aux anticorps, de la $T_3$ et/ou de la $T_4$ liée aux anticorps, ainsi que de la thyroglobuline libre et de la $T_3$ et/ou $T_4$ libre. La quantité de thyroglobuline liée aux anticorps dépend de la quantité de $T_4$ et/ou $T_3$ présente dans l'échantillon et cette quantité de thyroglobuline liée diminue lorsque la quantité de $T_4$ et/ou $T_3$ augmente. Aussi, pour doser la quantité de $T_4$ et/ou $T_3$ présente dans l'échantillon, on détermine la quantité de thyroglobuline liée aux anticorps ou la quantité de thyroglobuline sous forme libre.

Cette détermination peut être effectuée par les méthodes classiques employées dans les dosages immunologiques, méthodes qui font appel, soit à un marquage de l'un des composés utilisés pour la réaction de compétition, par exemple dans ce cas la thyroglobuline ou les anticorps anti-$T_3$ et/ou anti-$T_4$, soit à l'utilisation de composés additionnels marqués qui soient susceptibles de se fixer sur la thyroglobuline liée aux anticorps ou sur la thyroglobuline libre.

Généralement, ces composés additionnels sont constitués par des anticorps marqués spécifiques des $\gamma$-globulines de l'espèce ayant produit les anti-

corps anti-$T_3$ et/ou anti-$T_4$, par de l'anti-thyroglobuline marqué ou par des anticorps anti-$T_4$ et/ou des anticorps anti-$T_3$. On peut aussi utiliser comme composés additionnels des systèmes comprenant un composé marqué et une protéine, par exemple les systèmes anticorps biotinylés marqués-avidine en utilisant également dans le dosage des anti-corps anti-$T_3$ et/ou anti-$T_4$ biotinylés. Pour cette détermination, le marquage de la thyroglobuline ou des différents anticorps est réalisé par des méthodes classiques.

Aussi, dans la suite du présent texte, le terme "marqué" appliqué à la thyroglobuline ou à différents types d'anticorps, signifie que la thyroglobuline ou les anticorps ont été modifiés par un élément marqueur qui peut être par exemple un radioélément, un élément fluorescent, un élément luminescent, une enzyme, un chromophore fluorescent, un chromophore absorbant la lumière, la biotine, l'avidine ou la protéine A marquée au PAP (peroxydase-antiperoxydase).

Pour réaliser plus facilement cette détermination de la quantité de thyroglobuline liée aux anticorps ou de la thyroglobuline sous forme libre, on peut utiliser des méthodes classiques de dosage en phase hétérogène qui permettent de séparer la thyroglobuline liée aux anticorps de la thyroglobuline libre.

Toutefois, on peut aussi utiliser dans l'invention des méthodes de dosage en phase homogène qui ne comportent pas une telle séparation.

Lorsqu'on réalise le dosage en phase hétérogène, on utilise une phase solide sur laquelle est immobilisée soit la thyroglobuline, soit les anticorps anti-$T_3$ et/ou anti-$T_4$.

Les phases solides susceptibles d'être utilisées peuvent être de différents types. Ainsi, on peut utiliser des phases solides macroscopiques, constituées par des tubes, des billes ou des ailettes réalisés en polymère ou en d'autres matériaux.

Les polymères susceptibles d'être utilisés sont par exemple le polystyrène, les polyamides, le polypropylène, les polyoxyméthylène et les copolymères de styrène.

On peut aussi utiliser des phases solides microscopiques, finement divisées contenant de la thyroglobuline ou des anticorps anti-$T_3$ ou anti-$T_4$, par exemple des poudres, des agrégats, en polymère, en protéine ou en d'autres matériaux. De telles phases peuvent être constituées par de la thyroglobuline ou des anticorps anti-$T_3$ ou anti-$T_4$ rendus insolubles par des procédés physicochimiques ou immunologiques. Ces phases solides microscopiques peuvent être obtenues par exemple en formant dans le milieu réactionnel un précipité constitué par un réseau d'anticorps anti-IgG et IgG provenant de la même espèce que les anticorps anti-$T_3$ et/ou anti-$T_4$ utilisés pour le dosage, ce réseau étant capable de retenir les complexes thyroglobuline-anti-$T_3$, thyroglobuline-anti-$T_4$,   $T_3$-anti-$T_3$ et/ou $T_4$-anti-$T_4$. Ce réseau peut, par exemple, être formé d'antisérum de mouton anti-IgG de lapin et d'IgG de lapin dans le cas où les anticorps anti-$T_3$ et/ou anti-$T_4$ proviennent du lapin.

Dans le cas où l'on utilise des phases solides finement divisées, on peut séparer la phase solide du milieu liquide dans lequel a été effectuée la réaction de compétition par des techniques classiques, par exemple par centrifugation.

En revanche, dans le cas où l'on utilise les phases solides du type tube, billes ou ailettes, le processus de séparation est plus simple, en particulier lorsque ces phases solides sont solidaires des tubes où l'on effectue la réaction.

On décrit ci-après différents modes de réalisation du dosage de l'invention en phase hétérogène.

I. Dosages en phase hétérogène avec thyroglobuline immobilisée sur une phase solide.

Pour réaliser ces dosages, on fixe généralement la thyroglobuline sur les phases solides décrites précédemment en utilisant une solution de thyroglobuline, par exemple de thyroglobuline de boeuf ou de porc que l'on met en contact avec la phase solide pendant une durée suffisante qui peut aller d'une heure à une nuit, à diverses températures, afin de fixer la quantité voulue de thyroglobuline. Cette quantité dépend en particulier du temps de contact et de la température utilisés.

Ces phases solides sur lesquelles est immobilisée la thyroglobuline peuvent être utilisées pour doser séparément ou simultanément la triiodothyronine et la thyroxine.

Pour doser la thyroxine $T_4$ seule, on met en contact l'échantillon contenant la $T_4$ à doser avec la thyroglobuline immobilisée et une quantité limitée d'un anticorps anti-$T_4$, et on détermine ensuite la quantité d'anticorps anti-$T_4$ fixée à la thyroglobu-line immobilisée.

Pour doser la triiodothyronine $T_3$ seule, on met en contact l'échantillon contenant la triiodothyronine à doser avec la thyroglobuline immobilisée et une quantité limitée d'anticorps anti-$T_3$, et on dé-termine ensuite la quantité d'anticorps anti-$T_3$ fixés à la thyroglobuline immobilisée.

Dans les deux cas, on peut déterminer facilement la quantité d'anticorps anti-$T_4$ ou anti-$T_3$ fixés à la thyroglobuline immobilisée en utilisant comme anticorps des anticorps anti-$T_3$ et/ou anti-$T_4$ mono-clonaux ou polyclonaux marqués ou leurs fragments.

De préférence, on utilise comme marqueur une enzyme telle que le peroxydase de raifort, qui peut être couplée facilement aux anticorps utilisés et dont l'activité enzymatique peut être déterminée dans de bonnes conditions par des méthodes colorimétriques simples.

On peut aussi utiliser d'autres enzymes, par exemple la β-galactosidase ou une phosphatase alcaline.

Dans les deux dosages décrits ci-dessus, on peut aussi utiliser un second anticorps pour révéler la quantité d'anticorps anti-$T_3$ ou anti-$T_4$ fixés sur la thyroglobuline immobilisée, ou la quantité de thyroglobuline qui n'est pas liée aux anticorps anti-$T_3$ et/ou anti-$T_4$.

Dans ce cas, les anticorps anti-$T_3$ ou anti-$T_4$ constituent un premier anticorps et l'on utilise un second anticorps marqué capable de se fixer sur le premier anticorps ou sur la thyroglobuline. A titre d'exemple, le second anticorps peut être un anti-$T_3$ ou $T_4$ marqué qui viendra se fixer sur les sites $T_3$ ou $T_4$ de la thyroglobuline n'ayant pas réagi.

De cette façon la réaction de la $T_3$ ou de la $T_4$ avec les anticorps anti-$T_3$ ou anti-$T_4$ n'est pas perturbée puisqu'il n'est pas nécessaire de modifier chimiquement ces anticorps pour les marquer.

Toutefois, selon une variante de réalisation, adaptée à l'utilisation de marqueurs fluorescents, le premier anticorps peut être marqué par un chromophore fluorescent et le second anticorps par un chromophore absorbant la lumière de façon à détecter la quantité d'anticorps fixés sur la thyroglobuline par extinction de la réaction de fluorescence en utilisant la méthode décrite dans le brevet américain 4 174 384.

Pour doser simultanément la thyroxine $T_4$ et la triiodothyronine $T_3$, on met en contact l'échantillon à doser contenant $T_3$ et $T_4$ avec de la thyroglobuline et des quantités limitées d'anticorps anti-$T_3$ et d'anticorps anti-$T_4$ et on détermine ensuite la quantité d'anticorps anti-$T_3$ fixés à la thyroglobuline, et la quantité d'anticorps anti-$T_4$ fixés à la thyroglobuline.

Dans ce cas, on peut utiliser comme précédemment des anticorps anti-$T_3$ et des anticorps anti-$T_4$ marqués, mais il est nécessaire qu'ils soient marqués par des atomes et/ou des molécules différentes.

A titre d'exemple, les anticorps anti-$T_3$ peuvent être marqués par la β-galactosidase et les anticorps anti-$T_4$ peuvent être marqués par une phosphatase alcaline ou inversement, comme il est décrit par C. Blake et al. dans Clinical Chemistry, vol.28, n°7, (1982), p.1469-1473.

Dans ce dernier dosage, on peut aussi révéler les quantités d'anticorps anti-$T_3$ et anti-$T_4$ fixés sur la thyroglobuline en utilisant un second groupe d'anticorps marqués. Dans ce cas, les anticorps anti-$T_3$ et anti-$T_4$ introduits en quantité limitée constituent respectivement les premier et deuxième anticorps, et l'on utilise un troisième anticorps marqué capable de se fixer de façon spécifique sur le premier anticorps anti-$T_3$ et un quatrième anticorps marqué capable de se fixer de façon spécifique sur le deuxième anticorps anti-$T_4$, les troisième et quatrième anticorps étant marqués de façon différente.

Dans ce cas, les marqueurs utilisés peuvent être en particulier des enzymes, par exemple le couple β-galactosidase-phosphatase alcaline.

II. dosage en phase hétérogène avec anticorps immobilisés sur une phase solide

Dans ces dosages, les anticorps sont fixés sur une phase solide qui peut être constituée comme précédemment par une phase macroscopique ou microscopique en polymère ou en d'autres matériaux et les anticorps immobilisés peuvent être utilisés pour doser séparément ou simultanément la triiodothyronine et la thyroxine.

Dans ce cas, le mode de dosage est le même, mais pour pouvoir déterminer la quantité de thyroglobuline fixée aux anticorps anti-$T_3$ et/ou anti-$T_4$, il convient d'utiliser soit un second groupe d'anticorps marqués capables de se fixer spécifiquement sur un autre site réactif de la thyroglobuline, soit de la thyroglobuline marquée.

Lorsqu'on utilise un second groupe d'anticorps, ceux-ci peuvent être constitués par des anticorps anti-$T_3$ marqués et/ou anti-$T_4$ marqués, qui sont capables de se fixer spécifiquement sur un autre site réactif de la thyroglobuline.

Dans le premier cas qui est adapté au dosage immunologique de la thyroxine $T_4$, on met en contact la thyroxine à doser avec de la thyroglobuline, une quantité limitée d'anticorps anti-$T_4$ fixés sur une phase solide et des anticorps anti-$T_3$ et/ou anti-$T_4$ marqués, et on détermine ensuite la quantité d'anticorps anti-$T_3$ et/ou anti-$T_4$ marqués fixés sur le support solide par l'intermédiaire de la thyroglobuline.

Dans le cas où le procédé est destiné au dosage immunologique de la triiodothyronine $T_3$, on met en contact la triiodothyronine à doser avec de la thyroglobuline, une quantité limitée d'anticorps anti-$T_3$ fixés sur une phase solide et des anticorps anti-$T_4$ et/ou anti-$T_3$ marqués, et on détermine la quantité d'anticorps anti-$T_4$ et/ou anti-$T_3$ marqués fixés sur le support solide par l'intermédiaire de la thyroglobuline.

Lorsque l'on utilise de la thyroglobuline marquée, on met en contact l'échantillon liquide contenant la thyroxine $T_4$ (ou la triiodothyronine $T_3$) à doser avec de la thyroglobuline marquée et des anticorps anti-$T_4$ (ou anti-$T_3$ ) immobilisés sur un support solide et on détermine ensuite la quantité de thyroglobuline marquée fixée sur ledit support.

De préférence, on utilise de la thyroglobuline marquée par une enzyme qui peut être couplée à la thyroglobuline soit directement, soit par l'intermédiaire d'un anticorps anti-thyroglobuline, c'est-à-dire un composé conjugué thyroglobuline-anticorps anti-thyroglobuline-enzyme.

Les deux types de dosage en phase hétérogène décrits ci-dessus peuvent être effectués avec des ... ositifs classiques, en particulier avec des tubes à l'intérieur desquels est disposée une phase solide qui peut soit être la paroi du tube lui-même, soit être insérée dans celui-ci par exemple sous la forme d'un dispositif à ailettes comme il est décrit dans la demande de brevet européen EP-A-0 097 573 déposée le 9 juin 1983 au nom du C.E.A.

Pour ces dosages, on fixe tout d'abord sur la phase solide la thyroglobuline (ou les anticorps anti-$T_3$ et/ou anti-$T_4$), puis on introduit dans le tube l'échantillon liquide à doser et les anticorps anti-$T_3$ et/ou anti-$T_4$ (ou la thyroglobuline).

La $T_3$ et/ou la $T_4$ présente dans l'échantillon et la thyroglobuline entrent en compétition pour les sites anticorps et une certaine proportion d'anticorps vient se fixer sur la thyroglobuline immobilisée (ou une partie de la thyroglobuline vient se fixer sur les anticorps immobilisés). Après cette opération, on élimine du tube la phase liquide, puis on lave la phase solide et on détermine la quantité d'anticorps fixée sur la phase solide (ou la quantité de thyroglobuline fixée sur la phase solide) en utilisant les méthodes de révélation classiques qui ont été décrites précédemment, soit l'utilisation d'anticorps anti-$T_3$ et/ou anti-$T_4$ (ou de thyroglobuline) marqués, soit l'utilisation d'un second groupe d'anticorps marqués.

Lorsqu'on utilise un second groupe d'anticorps marqués, ceux-ci peuvent être ajoutés, soit pendant la réaction de compétition, soit après l'opération de lavage de la phase solide.

Pour chaque type de dosage, on effectue ces opérations sur des échantillons contenant des quantités connues de $T_3$ et/ou de $T_4$, afin d'établir une courbe d'étalonnage et on se reporte ensuite à cette courbe d'étalonnage pour déterminer la teneur en $T_3$ et/ou $T_4$ d'un échantillon.

L'invention a également pour objet une trousse pour réaliser un dosage en phase hétérogène de thyroxine $T_4$ utilisant un seul anticorps.

Cette trousse comprend :
- une série de tubes comportant chacun une phase solide revêtue dans les mêmes conditions de thyroglobuline,
- une série de flacons contenant des échantillons standards de thyroxine $T_4$, et
- un flacon contenant un anticorps anti-$T_4$ marqué.

De préférence, l'anticorps anti-$T_4$ marqué est constitué par un composé conjugué d'anticorps anti-$T_4$ monoclonal et d'une enzyme telle que la peroxydase de raifort.

Dans ce cas, la trousse comprend de plus :
- au moins un flacon contenant un chromogène pour la révélation enzymatique,
- un flacon contenant un tampon substrat pour la révélation enzymatique, et
- un flacon contenant un acide capable d'arrêter la réaction enzymatique.

Le chromogène utilisé pour la révélation enzymatique peut être le bis-chlorhydrate d'orthophénylène-diamine et l'acide capable d'arrêter la réaction enzymatique peut être l'acide oxalique.

A titre d'exemple, on donne ci-après la composition d'une trousse de ce type :
- 64 tubes ELSA comportant une phase solide du type ailette sur laquelle est fixée la thyroglobuline,
- 2 flacons de composé conjugué anticorps anti-$T_4$ monoclonal couplé à la peroxydase de raifort,
- 5 flacons d'échantillons standards de $T_4$ lyophilisés couvrant la gamme 0 à 50 pg/ml,
- 2 flacons de sérums de contrôle contenant de la $T_4$, lyophilisés,
- 2 flacons de chromogène lyophilisé, chaque flacon contenant 32 mg d'orthophénylènediamine-2HCl dans un milieu salin,
- 1 flacon de tampon substrat contenant 32 ml de tampon avec 0,02% d'$H_2O_2$ et un conservateur, et
- 2 sachets contenant chacun 3,15 g d'acide oxalique en poudre.

On peut réaliser une trousse du même type pour le dosage de la triiodothyronine $T_3$ en utilisant les mêmes constituants mis à part que l'on remplace l'anticorps anti-$T_4$ marqué par un anticorps anti-$T_3$ marqué, qui peut être également un composé conjugué d'anticorps anti-$T_3$ et d'une enzyme.

III. Dosages en phase homogène

Selon l'invention, on peut aussi réaliser le dosage de la thyroxine ou de la triiodothyronine en phase homogène en utilisant la même réaction de compétition entre la $T_3$ ou la $T_4$ à doser et la thyroglobuline. Dans ce cas, on peut utiliser par exemple trois modes opératoires différents.

Selon un premier mode opératoire, on utilise de la thyroglobuline marquée par une enzyme telle que la malate déshydrogénase qui présente la propriété d'avoir une activité enzymatique plus faible lorsqu'elle est couplée avec la thyroglobuline. En revanche, la fixation d'un anticorps anti-$T_4$ sur le complexe thyroglobuline-malate déshydrogénase rétablit partiellement l'activité initiale. De ce fait, on peut réaliser le dosage en phase homogène car l'activité enzymatique décroîtra en fonction de la quantité de $T_4$ ou $T_3$ présente dans l'échantillon à doser. Dans ce cas, on met en contact le composé conjugué thyroglobuline-malate déshydrogénase, l'anticorps anti-$T_4$ (ou anti-$T_3$) avec l'échantillon contenant l'hormone $T_4$ (ou $T_3$) à doser et le substrat permettant de déterminer l'activité enzymatique (acide malïque). On effectue ensuite directement dans le mélange réactionnel une mesure de cinétique en temps fixé par mesure à 340 nm correspondant à l'apparition de NADH.

Selon un second mode opératoire, on utilise de la thyrogobuline couplée à un phosphonate qui est capable d'inhiber de façon irréversible l'acétyl cholinestérase quand il n'est pas combiné à un anticorps anti-$T_4$ (ou anti-$T_3$). Dans ce cas, on met en contact l'échantillon à doser avec la thyroglobuline couplée au phosphonate et des anticorps anti-$T_4$ (ou anti-$T_3$) en quantité limitée. De la sorte, les complexes anti-$T_4$ (anti-$T_3$) thyroglobuline phosphonate sont en partie dissociés et la thyroglobuline phosphonate exerce son action inhibitrice. On détermine alors la quantité de thyroglobuline phosphonate fixée aux anticorps anti-$T_4$ (ou anti-$T_3$) en révélant par une méthode colorimétrique la quantité d'acétyl cholinestérase inhibée en utilisant comme substrat l'iodure d'acétyl $\beta$-(méthylthiocholine) et en effectuant la lecture à 415 nm.

Selon un troisième mode opératoire, on utilise de la thyroglobuline couplée à un fluorochrome tel que la fluorescéine dont la fluorescence augmente quand ce composé est couplé à l'anticorps correspondant. Dans ce cas, on met en contact l'échantillon à doser avec la thyroglobuline marquée à la fluorescéine et des anticorps anti-$T_4$ (ou anti-$T_3$) puis on détermine la polarisation de fluorescence du mélange réactionnel qui est inversement proportionnel à la quantité de $T_4$ (ou $T_3$) à doser fixée au composé thyroglobuline-fluorescéine.

Les anticorps anti-$T_3$ et les anticorps anti-$T_4$ utilisés dans le procédé de l'invention peuvent être des anticorps polyclonaux ou des anticorps monoclonaux.

Les anticorps polyclonaux peuvent être obtenus par hyperimmunisation de moutons ou de lapins à l'aide de composés conjugués de $T_3$ ou $T_4$ avec de la sérumalbumine de boeuf préparés par exemple par la méthode au carbodiimide.

On recueille les immunoglobulines de l'antisérum par précipitation à l'acide caprylique selon la méthode décrite par Steinbuch et al. dans C.R. Soc. Biol. Paris 164, 296-301, 1970. Les anticorps polyclonaux peuvent être ensuite marqués par une enzyme telle que la peroxydase de raifort en utilisant la méthode au periodate de sodium décrite par Wilson M.B. et Nakane P.K. dans W.Knapp, K. Holubar, G. Wick (Eds.). Elsevier/north Holland Biomedical Press, 1978, pp.215-224.

On peut aussi préparer la fraction Fab' des immunoglobulines de l'antisérum en soumettant les IgG purifiées à une digestion pepsique suivie d'une réduction par la 2-mercaptoéthylamine. La fraction Fab' ainsi produite peut être ensuite marquée par une enzyme telle que la peroxydase de raifort en utilisant la méthode au maléimide décrite par Ishikawa et al. - J. Immunoassay, 1983, 4/3.

Lorsqu'on utilise des anticorps monoclonaux, ceux-ci peuvent être produits en utilisant la technique décrite par Köhler G. et Milstein C. dans Nature 256, pp.495-497, 1975. Cette technique consiste pour l'essentiel à injecter à une souris ou à un autre animal convenable de la thyroxine ou de la triiodothyronine, par exemple sous la forme d'un composé conjugué $T_3$ ou $T_4$ -sérumalbumine de boeuf. La souris est ensuite sacrifiée et des cellules prélevées sur sa rate sont fusionnées à des cellules de myélome. Il en résulte une cellule hybride appelée "hybridome" qui se reproduit in vitro. La population d'hybridomes est sélectionnée et manipulée de manière à isoler des clones individuels dont chacun élabore une espèce unique d'anticorps relative à la thyroxine ou à la triiodothyronine injectée.

D'autres caractéristiques et avantages de l'invention apparaîtront mieux à la lecture des exemples suivants, donnés bien entendu à titre illustratif, en référence au dessin annexé sur lequel les figures 1 et 2 sont des courbes illustrant le dosage de la thyroxine $T_4$ par le procédé de l'invention, les figures 3 et 4 sont des courbes illustrant le dosage de la triiodothyronine $T_3$ par le procédé de l'invention, la figure 5 est une courbe illustrant le dosage de la $T_4$ par le procédé de l'invention et la figure 6 est une courbe illustrant un dosage de $T_4$ utilisant de la thyroglobuline marquée.

Exemple 1 : Dosage de la thyroxine $T_4$ totale

1° Préparation des réactifs.

a) Préparation du support solide contenant la thyroglobuline.

On utilise de la thyroglobuline de boeuf ou de porc, produite ou purifiée à partir de la glande thyroïde selon le procédé de "Proceedings of the Society for Experimental Biology an Medicine", 29, (1931-32), qui est dissoute à raison de 5mg.ml$^{-1}$ dans un tampon phosphate de sodium (0,1 mol.$^{-1}$; pH 7,2), et conservée à -20°C. Le support solide est constitué par un dispositif à ailettes en polyamide ayant la forme d'une hélice à 5 pales de 2,5 cm$^2$ de surface qui est placé dans un tube à fond plat tel que celui décrit dans le brevet européen EP-A-0 097 573 du C.E.A.

Pour fixer la thyroglobuline on dilue tout d'abord la solution de thyroglobuline au moyen du même tampon phosphate pour obtenir une concentration de 0,025 mg.ml⁻¹ et on remplit le tube de cette solution. Après l'avoir maintenue à la température ambiante pendant une nuit, on retire la solution et on lave plusieurs fois le dispositif à ailettes avec le même tampon phosphate (0,1 mol.l⁻¹ ; pH 7,2) et on le conserve à 4°C dans ce même tampon.

Dans le cas où l'on utilise de la thyroglobuline de boeuf ou de porc, la quantité fixée sur l'ailette est de 2 μg.

b) Préparation des anticorps anti-T₄ couplés à une enzyme constituée par la peroxydase de raifort.

On prépare tout d'abord un composé conjugué immunogène thyroxine-sérumalbumine de boeuf (SAB) par une réaction catalysée par la carbodiimide entre les groupements carboxylique et/ou amine de la thyroxine et les groupements amine et/ou carboxylique de la sérumalbumine de boeuf.

Dans ce but, on dissout 20mg de T₄ dans 20ml de NaOH à 0,01 mol.l⁻¹, on ajoute 10% de diméthylformamide, puis on mélange cette solution avec 40mg de sérumalbumine de boeuf. Après dissolution, on ajoute 80 mg de 1-éthyl-3(3-diméthylamino-propyl) carbodiimide (EDCI) et on ajuste le pH à 8,5 avec HCl 0,5 N. On laisse incuber le mélange pendant 10 à 12h à la température ambiante, puis on le dialyse une nuit contre une solution de NaCl à 140 mmol.l⁻¹. On obtient ainsi un composé conjugué thyroxine sérum albumine de boeuf que l'on lyophilise et conserve à -20°C.

On immunise des moutons par injections intradermiques de 1 mg de ce composé conjugué après l'avoir émulsifié dans l'adjuvant complet de Freund. On répète ces injections tous les mois pendant 8 mois et on prélève le sang par ponction dans la veine jugulaire. On évalue le titre de l'antisérum par dosage radioimmunologique. Ce titre est défini comme étant la dilution finale capable de lier 50% de l'activité d'un traceur T₄I¹²⁵. On obtient un antisérum ayant un titre de 1/50 000 et une constante d'affinité de 5.10⁻⁹ mol⁻¹.

On prépare une fraction enrichie en immunoglobuline IgG à partir du sérum de mouton anti-T₄ selon la méthode à l'acide caprylique de Steinbuch et Audran, Archives of Biochemistry and Biophysis, 1979, 134, 279-284.

On ajoute à 10 ml de sérum, 20 ml de tampon acétate de sodium (0,06 mol.l⁻¹ ; pH 4) et 0,68 ml d'acide caprylique (pureté 96%), en ajoutant l'acide caprylique goutte à goutte et sous agitation constante. On maintient l'agitation pendant 30 min. à la température ambiante et on centrifuge le mélange pendant 10 min. à 10000g. On filtre le surnageant qui contient les IgG sur papier, puis on dialyse une nuit à 4°C contre un tampon phosphate de sodium (17,5 mmol.l⁻¹ ; pH 6,5). On recueille ainsi 400 mg d'IgG que l'on purifie par passage sur une colonne de cellulose équilibrée avec un tampon phosphate de sodium (17,5 mmol.l⁻¹) ; pH 6,5). On vérifie la pureté de la fraction d'IgG recueillie par électrophorèse contre un immunosérum de lapin antiprotéines de mouton. On obtient ainsi des IgG purifiées ayant un degré de pureté supérieur à 90%.

A partir de cette fraction d'IgG on forme le composé conjugué anti-T₄ peroxydase de raifort en utilisant la méthode au periodate de sodium décrite par M.B. Wilson et P.K. NAKANE dans W. Knapp, K. HOLUBAR et G. WICK (Eds), Immunofluorescence and related staining techniques Elseviet/North Holland Biomedical Press, 1978, p.215-224.

Après la réaction de couplage, on réduit par NaBH₄ et on purifie le composé conjugué par précipitation au sulfate d'ammonium (NH₄)₂SO₄ et on le conserve en fractions aliquotes à -20°C avec 1% de sérumalbumine de boeuf.

Lors de l'utilisation, on dilue l'IgG anti-T₄ marquée à la peroxydase de raifort dans un mélange contenant 1% de sérumalbumine de boeuf et de l'ANS (0,04%) dans du tampon phosphate (0,1 mol.l⁻¹ ; pH 7,2).

c) Préparation des échantillons standard.

Pour préparer les échantillons standard, on dissout de la thyroxine T₄ à une concentration de 1 mg/ml dans du NaOH, 0,01 N. On dilue ensuite les échantillons pour obtenir les concentrations désirées en utilisant pour ces dilutions un pool de sérum humain débarrassé de T₄ par traitement au charbon à raison de 100 mg/ml⁻¹ pendant une nuit à +4°C, suivi de deux centrifugations et d'une filtration sur un tamis de 0,22 μm.

2° Dosage enzymoimmunologique (T₄ totale).

On introduit dans des tubes à fond plat comportant chacun un dispositif à ailettes revêtu de thyroglobuline de porc des volumes de 10 ou 20 microlitres d'échantillons standards ayant respectivement des concentrations en T₄ de 0 ; 25 ; 50 ; 125 ; 250 μg/l⁻¹ et 20 mg/dl⁻¹ (blanc). On introduit ensuite dans chacun des tubes 0,5 ml de IgG anti-T₄ marquée à la peroxydase de raifort après l'avoir dilué à 20 μg/ml dans le diluant décrit précédemment. On laisse incuber pendant une heure à la température ambiante, puis on aspire la phase liquide présente dans chacun des tubes et on lave les dispositifs à ailettes deux fois avec de l'eau distillée contenant 0,05% de Tween® 20. On détermine alors l'activité enzymatique liée aux ailettes en la révélant par addition de 0,5 ml de la solution substrat constituée par du tampon phosphate citrate de sodium (0,1 mol.l⁻¹ ; pH 5,5) contenant 5,5 mmol.l⁻¹ d'eau oxygénée et 3g.l⁻¹ de bichlorhydrate d'orthophénylène diamine (pH final 5± 0,1) et on maintient les tubes à la température ambiante et à l'obscurité pendant 30 min. On arrête alors la réaction par addition de 1 à 2 ml de HCl 1N et on mesure les absorbances à 492 nm.

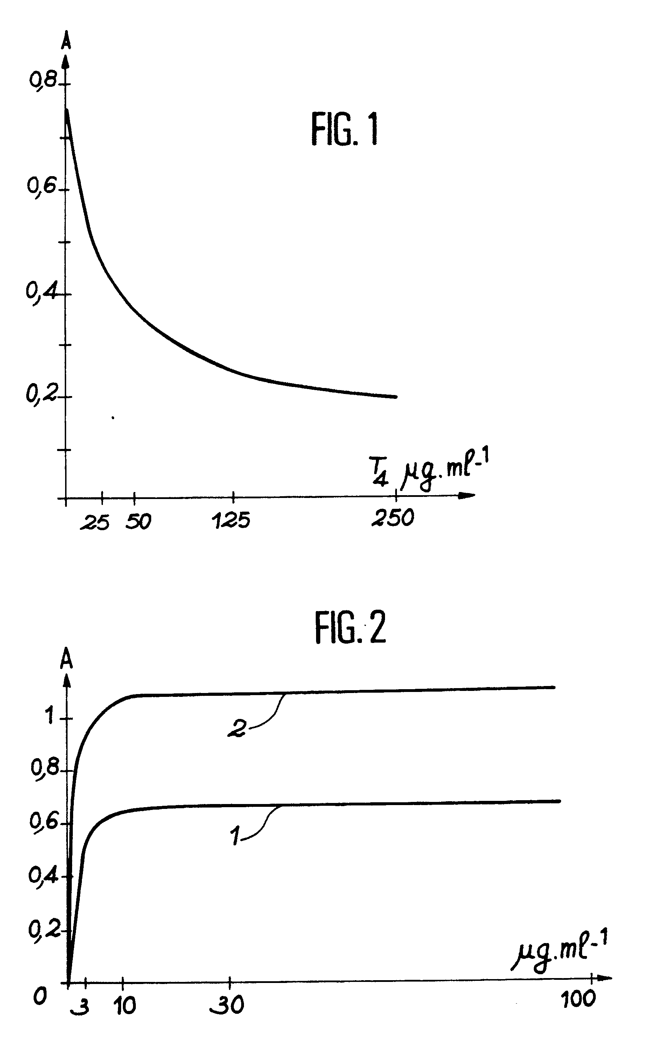

Les résultats obtenus sont donnés sur la figure 1 qui représente l'évolution de l'absorbance à 492 nm en fonction de la concentration en $T_4$ des échantillons, et correspond à la courbe d'étalonnage du dosage. A l'aide de cette courbe on détermine la concentration en $T_4$ de l'échantillon à doser.

Dans cet exemple, on a optimisé le dosage en effectuant les mêmes opérations sur des échantillons standard à 0ng.ml⁻¹ de $T_4$ en utilisant des dispositifs à ailettes ayant des concentrations différentes en thyroglobuline de porc ou de boeuf. Ces concentrations différentes sont obtenues en préparant le dispositif à ailettes selon le même mode opératoire que celui décrit ci-dessus en 1.a) mais en utilisant des concentrations différentes en thyroglobuline de la solution de fixation.

Les résultats obtenus sont donnés sur la figure 2 qui représente l'absorbance à 492 nm en fonction de la concentration en thyroglobuline ($\mu$g.ml⁻¹) de la solution utilisée pour la préparation du support solide. La courbe 1 illustre les résultats obtenus avec la thyroglobuline de boeuf et la courbe 2 illustre les résultats obtenus avec la thyroglobuline de porc.

Au vu de cette figure, on remarque que le plateau d'absorption est obtenu pour des concentrations voisines de 0,01mg.ml⁻¹ dans les deux cas.

Au vu des résultats donnés sur la figure 2, on constate que la thyroglobuline de porc a plus de capacité de fixation d'anticorps anti-$T_4$ que celle de boeuf.

Si l'on répète ces opérations en utilisant dans les échantillons standard de la thyroglobuline de boeuf ou de la thyroglobuline de porc au lieu de $T_4$, on peut calculer les équivalents réactionnels en thyroxine $T_4$ de ces deux thyroglobulines. Ainsi, on trouve que la thyroglobuline de boeuf contient 700 ng de $T_4$ pour 5 mg de thyroglobuline, soit 0,14 mol de $T_4$ par mol de thyroglobuline.

Dans le cas de la thyroglobuline de porc, celle-ci contient l'équivalent de 0,6 mol de $T_4$ par mol de thyroglobuline.

Exemple 2 : Dosage de la triiodothyronine $T_3$ totale.

Dans cet exemple, on suit le même mode opératoire que dans l'exemple 1, pour déterminer la concentration en triiodothyronine d'échantillons, en utilisant comme anticorps anti-$T_3$ des IgG anti-$T_3$ couplés à la peroxydase de raifort obtenus de la même façon que les IgG anti-$T_4$ couplés à la peroxydase de raifort.

Ce support solide est constitué par des billes de polystyrène revêtues de thyroglobuline de boeuf par immersion pendant une heure à 45°C dans un tampon phosphate de sodium (0,1 mol.l⁻¹ ; pH 7,2) contenant 0,1 mg.ml⁻¹ de thyroglobuline de boeuf. Après plusieurs lavages au moyen du même tampon, on conserve les billes à +4°C dans le même tampon contenant 0,01% de Thimérosal. Avant utilisation, on sature les billes avec une solution de BSA à 1% dans du PBS (pH 7,2 ; 0,1 mol.l⁻¹) pendant une demi-heure.

On utilise les mêmes diluants et le même mode de préparation des échantillons standard.

Pour le dosage, on introduit dans des cupules 0,1 ml d'échantillon standard ou d'échantillon à doser et 0,3 ml d'IgG anti-$T_3$ marquée à la peroxydase de raifort diluée au 1/2000 dans le diluant, ce qui correspond à une dilution de 1/20000 de l'antisérum initial, ainsi que les billes revêtues de thyroglobuline de boeuf. On laisse incuber pendant 2h à la température ambiante puis on extrait les billes, on les lave 3 fois avec 5 ml d'eau distillée, et on les transfère dans des tubes en polystyrène jetable. On détermine alors l'activité enzymatique des billes utilisant le même mode opératoire que dans l'exemple 1 mais en utilisant seulement 0,3 ml de la solution substrat. Comme dans l'exemple 1, la réaction est arrêtée au bout d'une demi-heure par addition de 1ml de HCL 1N.

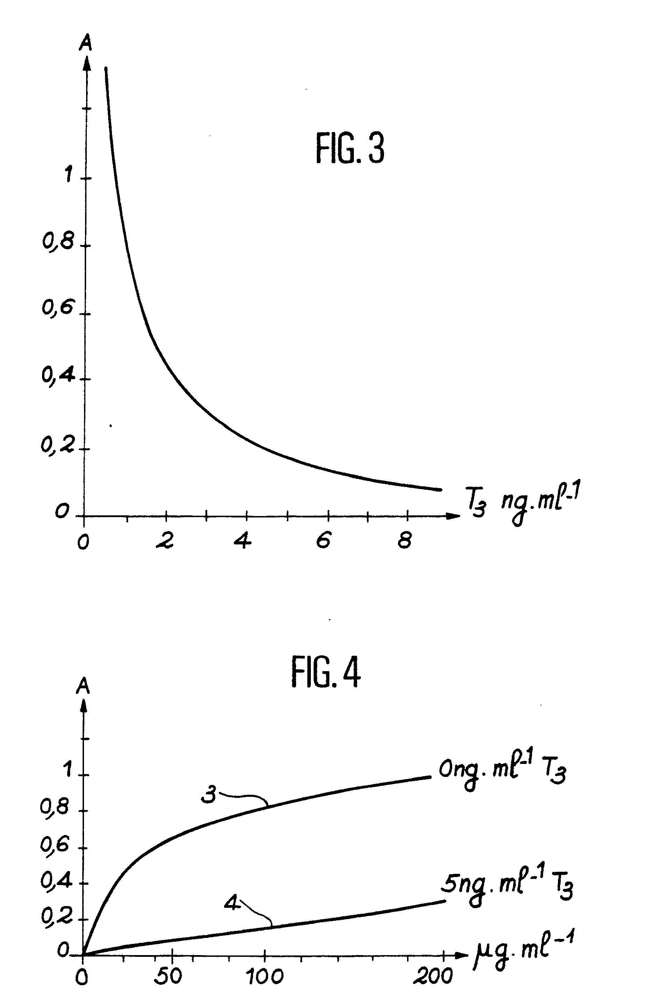

Les résultats obtenus pour divers échantillons standards sont donnés sur la figure 3 qui représente l'absorbance à 492 nm en fonction de la concentration en $T_3$ (en ng.ml⁻¹) ce qui correspond à la courbe d'étalonnage en $T_3$.

A l'aide de cette courbe, on peut déterminer la concentration en $T_3$ de l'échantillon à doser.

Dans cet exemple, on a optimisé le dosage en effectuant les mêmes opérations avec des échantillons standards contenant soit 0 ng.ml⁻¹ de $T_3$, soit 5 ng.ml⁻¹ de $T_3$ en utilisant des billes de polystyrène ayant des concentrations différentes en thyroglobuline de boeuf. Les résultats obtenus sont donnés sur la figure 4 où la courbe 3 représente les variations de l'absorbance des échantillons à 0 ng.ml⁻¹ de $T_3$ en fonction de la concentration en thyroglobuline de boeuf de la solution utilisée pour la préparation des billes (en $\mu$g.ml⁻¹, et la courbe 4 représente l'absorbance des échantillons contenant 5 ng.ml⁻¹ de $T_3$ en fonction de la concentration en thyroglobuline de boeuf de la solution utilisée pour la préparation des billes (en $\mu$g.ml⁻¹).

On répète encore ces opérations comme dans l'exemple 1 en utilisant des échantillons standards contenant de la thyroglobuline de boeuf au lieu de triiodothyronine, et on détermine ainsi à partir de la courbe d'étalonnage de la figure 3, l'équivalent réactionnel en triiodothyronine de la thyroglobuline de boeuf. On trouve ainsi que la thyroglobuline de boeuf contient l'équivalent de 0,06mole de T$_3$ par mole de thyroglobuline de boeuf.

On répète le même mode opératoire en utilisant un support solide constitué par des billes de polystyrène recouvertes de thyroglobuline de porc. Les résultats obtenus dans ces conditions sont équivalents.

Exemple 3 : Dosage de la thyroxine T$_4$ libre.

Dans cet exemple, on suit le même mode opératoire que dans l'exemple 1 en utilisant des ailettes en polypropylène recouvertes de thyroglobuline de porc. Les échantillons standards sont préparés comme dans le cas de l'exemple 1 dans une gamme de 0 à 50 pg de T$_4$ libre par ml. L'étalonnage en T$_4$ libre est fait par une technique de référence.

Le composé conjugué anti-T$_4$/enzyme est formé à partir de la fraction Fab' de l'antisérum produit en immunisant des moutons comme dans l'exemple 1. Cette fraction Fab' est obtenue à partir de l'IgG produite dans l'exemple 1 par digestion pepsique suivie d'une réduction à l'aide de 2-mercapto-éthylamine. Pour réaliser la digestion pepsique, on ajoute 0.125 ml de NaCl à 2 mol.l$^{-1}$ et 2 mg de pepsine à 2,5 ml d'IgG à 20 mg.ml$^{-1}$. On laisse incuber le mélange pendant 24h à 37°C et on arrête la digestion par addition de NaOH 1N jusqu'à l'obtention d'un pH de 8. Après centrifugation, on filtre le surnageant sur une colonne Ultrogel® ACA44 et on élue les fragments par un tampon phosphate de sodium (0,1 mol.l$^{-1}$ ; pH 6). On recueille ainsi 12 mg de Fab'$_2$ dans 20 ml que l'on concentre 10 fois avec du polyéthylène glycol de masse moléculaire 35000. On réduit ensuite la fraction ainsi obtenue en Fab' en ajoutant 0,22 ml de 2-mercaptoéthylamine à 0,1 mol.l$^{-1}$ dans un tampon phosphate de sodium (0,1 mol.l$^{-1}$)-acide éthylène-diaminetétraacétique (EDTA) (5 mmol.l$^{-1}$) ayant un pH de 6. On laisse incuber le mélange pendant 1h30 à 37°C, puis on filtre la solution sur colonne Séphadex® G25 avec le même tampon phosphate de sodium-EDTA. On obtient ainsi 10,5 mg de la fraction Fab'.

Cette fraction d'anticorps est alors marquée par la peroxydase de raifort de la façon suivante.

On dissout tout d'abord 14 mg de peroxydase de raifort dans 2,1 ml de tampon phosphate de sodium (0,1 mol.l$^{-1}$ ; pH 7). On dissout séparément 7.5 mg de N-succinimidyl-4-(N-maléimidométhyl) cyclohexane-1-carboxylate dans 0,18ml de diméthylformamide. On mélange ensuite les deux solutions et on les laisse incuber sous agitation pendant 45 min. à 30°C. Après centrifugation, on filtre le surnageant sur une colonne de Séphadex® G25 et on élue avec un tampon phosphate de sodium (0,1 mol.l$^{-1}$ ; pH 6) ce qui permet d'obtenir une solution de peroxydase maléimide ayant une concentration en peroxydase de raifort de 5,2 mg.ml$^{-1}$. On mélange alors 10 mg de cette solution dans 1 ml de tampon phosphate de sodium (0,1 mol.l$^{-1}$ ; pH 6) avec 10,5 mg de fragments Fab' dans 1 ml de tampon EDTA (5 mmol.l$^{-1}$) phosphate de sodium (0,1 mol.l$^{-1}$) (pH 6) et on laisse incuber pendant une heure à 30°C. On filtre alors le composé conjugué Fab'-peroxydase de raifort sur une colonne d'Ultrogel® et on élue avec un tampon phosphate de sodium (0,1 mol.l$^{-1}$ ; pH 6,5). Le premier pic correspond au conjugué qui a un poids moléculaire de 100000. (Volume d'élution : 90 ml).

Pour réaliser le dosage, on introduit dans des tubes contenant les ailettes de polypropylène recouvertes de thyroglobuline de porc, 0,05 ml d'échantillon standard ou d'échantillon à doser et 0,5 ml du conjugué Fab' anti-T$_4$ peroxydase de raifort dilué au 300 ng.ml$^{-1}$ dans un tampon phosphate de sodium (0,1 mol.l$^{-1}$ ; pH 7,2) contenant 0,1% de sérumalbumine de boeuf. On laisse incuber pendant 3h à 37°C, puis on élimine le liquide des tubes et on lave deux fois les ailettes à l'eau distillée. On détermine alors l'activité enzymatique des ailettes en ajoutant 0,5 ml de la solution substrat utilisée dans l'exemple 1 et on laisse incuber pendant 30 min. à la température ambiante. On arrête alors la réaction par addition d'1 ml d'acide oxalique 1N et on détermine la densité optique à 492 nm.

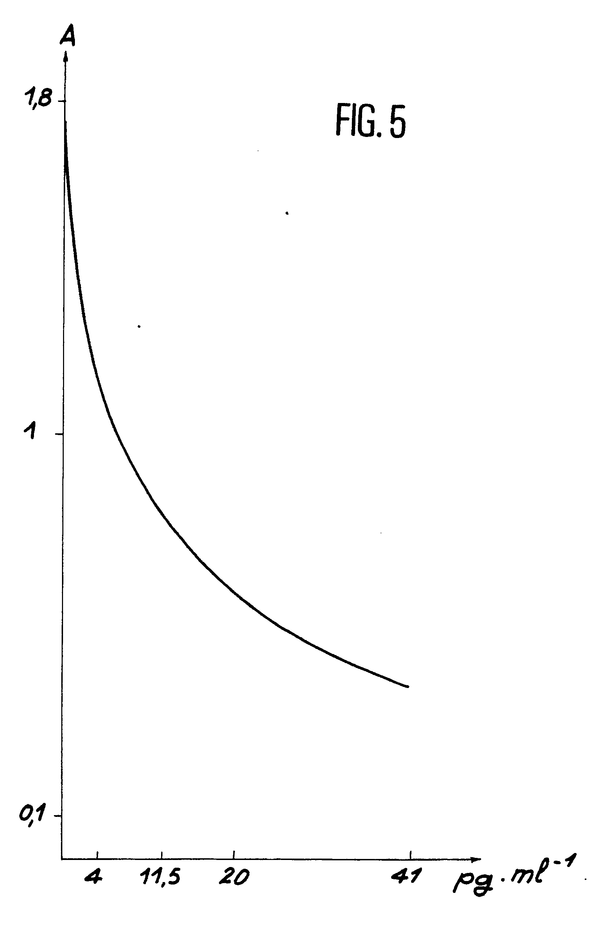

Les résultats obtenus sont donnés sur la figure 5 qui correspond à la courbe d'étalonnage du dosage.

Exemple 4 : Dosage de la thyroxine T$_4$ totale.

Pour ce dosage, on suit sensiblement le même mode opératoire que dans l'exemple 3 mais en utilisant des billes de polystyrène recouvertes de thyroglobuline de boeuf et les anticorps anti-T$_4$ marqués à la peroxydase de raifort de l'exemple 1. Pour le dosage, on introduit dans des tubes contenant les billes de polystyrène 0,05 ml d'échantillon standard ou d'échantillon à doser et 0,3 ml du composé conjugué anti-T$_4$ peroxydase de raifort dilué au 20 μg/ml dans un tampon phosphate de sodium (0,1 mol.l$^{-1}$ ; pH 7,2) contenant 1% de SAB et 0,04% de ANS. On laisse incuber pendant 1h à la température ambiante. On sépare le liquide des billes et on lave celles-ci deux fois à l'eau distillée. On détermine alors l'activité enzymatique par addition de 0,3 ml du substrat utilisé dans l'exemple 1 et on laisse incuber pendant 30 min à la température ambiante. On arrête alors la réaction par addition d'1 cm$^3$ d'acide oxalique 1N et on détermine la densité optique à 492 nm.

Exemple 5 : Dosage de la thyroxine $T_4$ totale.

Dans cet exemple, on réalise le dosage en phase hétérogène en utilisant de la thyroglobuline marquée par une enzyme et des anticorps anti-$T_4$ immobilisés sur un support solide réalisé en polypropylène.

a) Préparation des anticorps anti-$T_4$ immobilisés.

On fixe tout d'abord les anticorps anti-$T_4$ qui ont été préparés de la même manière que dans l'exemple 1, sur les supports en polypropylène en immergeant 100 supports dans 100 ml d'une solution d'anti-$T_4$ contenant 0,03 mg/ml d'IgG anti-$T_4$ en agitant le mélange réactionnel pendant 18h à la température ambiante. On lave ensuite les supports avec 1l de PBS et on les recouvre avec de la BSA à 0,1% dans du PBS pendant 4h à la température ambiante. On les rince ensuite à l'eau distillée, on les lyophilise puis on les stocke dans des bouteilles en polyéthylène à la température ambiante.

b) Préparation de la thyroglobuline marquée.

On prépare ensuite de la thyroglobuline marquée à la peroxydase de raifort de la façon suivante. On active 100mg de peroxydase de raifort avec du glutaraldéhyde à 1% en utilisant la méthode d'Avrameas et Ternynck décrite dans Immunochemistry, vol.8, p.1175 (1971). On fait passer ensuite la peroxydase de raifort activée au glutaraldéhyde sur une colonne de Séphadex® G25 (2,5×20 cm) équilibrée avec une solution saline et on recueille les fractions qui ne sont pas de couleur brune. On ajoute à 4,5ml de la solution de peroxydase de raifort activée 1ml de solution de carbonate bicarbonate (1 M ; pH 9,5), puis on ajoute goutte à goutte à la solution sous agitation 3 mg de thyroglobuline dissoute dans une solution de carbonate bicarbonate 1M, pH 9,5. On agite le mélange réactionnel pendant 5h à la température ambiante et on le laisse pendant une nuit à 4°C. On concentre ensuite la solution de composé conjugué thyroglobuline-péroxydase de raifort à 2 ml par dialyse contre du polyéthylène glycol PEG 35000 sec et on purifie le conjugué par filtration sur Sepharose® 4B.

On purifie ensuite le composé conjugué sur une colonne de Sépharose® en réalisant l'élution avec un gradient discontinu allant de 0 à 50% d'éthylène glycol dans une solution saline. On vérifie le profil d'élution en déterminant l'activité enzymatique. Les pics contenant le composé conjugué thyroglobuline-peroxydase de raifort sont stabilisés par addition de 1% de BSA et 0,01% de thimérosal mélangés avec un volume égal de glycérol et stockés à -20°C.

c) Préparation des échantillons standard de $T_4$.

On prépare ceux-ci en utilisant le même mode opératoire que dans l'exemple 1.

d) Dosage enzymoimmunologique.

On introduit dans des tubes un polystyrène contenant les supports en polypropylène revêtus d'anticorps anti-$T_4$, 0,025 ml d'échantillon standard et 0,5 ml du conjugué thyroglobuline-peroxydase de raifort dilué dans PB à 1% BSA et 0,04% de l'ANS. On laisse incuber pendant une heure à la température ambiante, puis on vide les tubes et on lave deux fois les supports en polypropylène avec 5ml d'eau distillée contenant 0,05% de Tween® 20. On introduit alors dans chaque tube 0,5ml du substrat fraîchement préparé utilisé dans l'exemple 1 et on maintient les tubes à la température ambiante et dans l'obscurité pendant 30 min. On arrête alors la réaction enzymatique par addition de 1ml d'acide oxalique 1N et on mesure les absorbances à 492 nm contre un blanc qui correspond à un échantillon standard ayant une concentration en $T_4$ de 200 mg par litre.

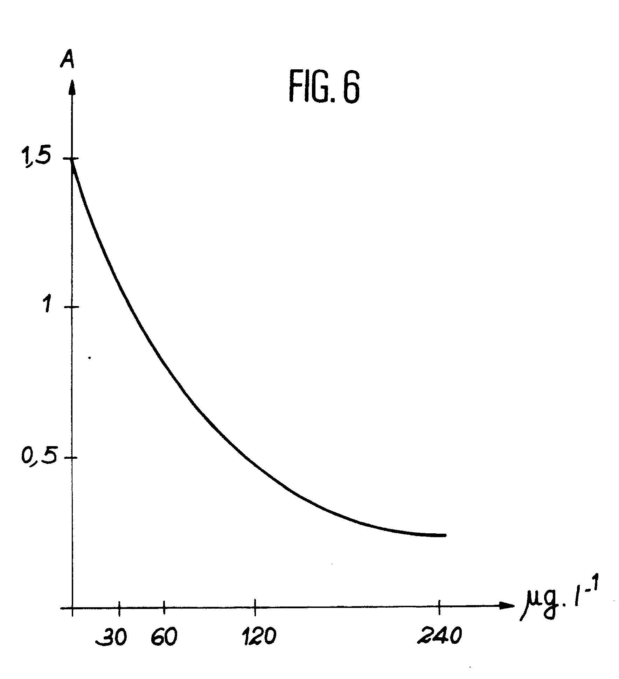

Les résultats obtenus avec l'échantillon standard sont donnés sur la figure 6 qui représente l'absorbance à 492 nm en fonction de la concentration en $T_4$ (en $\mu$g/l) standard, ce qui donne la courbe d'étalonnage en $T_4$.

A l'aide de cette courbe, on peut déterminer ensuite la concentration en $T_4$ de l'échantillon à doser en effectuant les mêmes opérations que précédemment, en déterminant l'absorbance à 492 nm et en se reportant à la courbe d'étalonnage pour trouver la concentration en $T_4$ correspondant à cette absorbance.

## Revendications

1. Procédé de dosage immunologique de la thyroxine $T_4$ et/ou la triiodothyronine $T_3$ présentes sous forme libre dans un échantillon, caractérisé en ce qu'il consiste à mettre en compétition $T_3$ et/ou $T_4$ à doser avec de la thyroglobuline pour les sites d'anticorps anti-$T_3$ et/ou anti-$T_4$ présents en quantité limitée et à déterminer ensuite, soit la quantité de thyroglobuline fixée aux anticorps anti-$T_3$ et/ou anti-$T_4$, soit la quantité de thyroglobuline non fixée aux anticorps anti-$T_3$ et/ou anti-$T_4$.

2. Procédé selon la revendication 1, caractérisé en ce que la thyroglobuline est immobilisée sur une phase solide.

3. Procédé selon la revendication 2, caractérisé en ce que la phase solide contenant la thyroglobuline immobilisée est constituée par de la thyroglobuline rendue insoluble par un procédé physicochimique ou immunologique.

4. Procédé de dosage immunologique de la thyroxine $T_4$ selon la revendication 2, caractérisé en ce qu'il consiste à mettre en contact l'échantillon contenant la $T_4$ à doser avec la thyroglobuline immobilisée et une quantité limitée d'anticorps anti-$T_4$ et à déterminer ensuite la quantité d'anticorps anti-$T_4$ fixés à la thyroglobuline immobilisée.

5. Procédé de dosage immunologique de la triiodothyronine $T_3$ selon la revendication 2, caractérisé en ce qu'il consiste à mettre en contact l'échantillon contenant la triiodothyronine à doser $T_3$ avec la thyroglobuline immobilisée et une quantité limitée d'anticorps anti-$T_3$ et à déterminer ensuite la quantité d'anticorps anti-$T_3$ fixés à la thyroglobuline immobilisée.

6. Procédé de dosage immunologique de la thyroxine $T_4$ et de la triiodothyronine $T_3$ selon la revendication 2, caractérisé en ce qu'il consiste à mettre en contact l'échantillon à doser contenant $T_3$ et $T_4$ avec la thyroglobuline immobilisée et une quantité limitée d'anticorps anti-$T_3$ et d'anticorps anti-$T_4$ et à déterminer ensuite la quantité d'anticorps anti-$T_3$ fixés à la thyroglobuline et la quantité d'anticorps anti-$T_4$ fixés à la thyroglobuline.

7. Procédé selon l'une quelconque des revendications 4 et 5, caractérisé en ce que les anticorps anti-$T_3$ et/ou les anticorps anti-$T_4$ sont des anticorps marqués.

8. Procédé selon l'une quelconque des revendications 4 et 5, caractérisé en ce que les anticorps anti-$T_3$ ou anti-$T_4$ constituent un premier anticorps et en ce que l'on utilise un second anticorps marqué capable de se fixer sur le premier anticorps.

9. Procédé selon l'une quelconque des revendications 4 et 5, caractérisé en ce que les anticorps anti-$T_3$ ou anti-$T_4$ constituent un premier anticorps, en ce que l'on utilise un second anticorps capable de se fixer sur le premier anticorps et en ce que le premier anticorps est marqué par un chromophore fluorescent et le second anticorps par un chromophore absorbant la lumière.

10. Procédé selon la revendication 6, caractérisé en ce que les anticorps anti-$T_3$ et les anticorps anti-$T_4$ sont marqués par des atomes et/ou des molécules différents.

11. Procédé selon la revendication 10, caractérisé en ce que les anticorps anti-$T_3$ et les anticorps anti-$T_4$ sont marqués par deux enzymes différentes.

12. Procédé selon la revendication 6, caractérisé en ce que les anticorps anti-$T_3$ constituent un premier anticorps et les anticorps anti-$T_4$ constituent un deuxième anticorps, et en ce que l'on utilise un troisième anticorps marqué capable de se fixer de façon spécifique sur le premier anticorps et un quatrième anticorps marqué capable de se fixer de façon spécifique sur le deuxième anticorps, les troisième et quatrième anticorps étant marqués de façon différente.

13. Procédé de dosage immunologique de la thyroxine $T_4$ selon la revendication 1, caractérisé en ce qu'il consiste à mettre en contact la thyroxine à doser avec de la thyroglobuline, une quantité limitée d'anticorps anti-$T_4$ fixés sur une phase solide et des anticorps anti-$T_3$ ou anti-$T_4$ marqués, et à déterminer ensuite la quantité d'anticorps anti-$T_3$ ou anti-$T_4$ marqués fixés sur le support solide par l'intermédiaire de la thyroglobuline.

14. Procédé de dosage immunologique de la triiodothyronine $T_3$ selon la revendication 1, caractérisé en ce qu'il consiste à mettre en contact la triiodothyronine à doser avec de la thyroglobuline, une quantité limitée d'anticorps anti-$T_3$ fixés sur une phase solide, et des anticorps anti-$T_4$ ou anti-$T_3$ marqués, et à déterminer la quantité d'anticorps anti-$T_4$ u anti-$T_3$ marqués fixés sur le support solide par l'intermédiaire de la thyroglobuline.

15. Procédé selon l'une quelconque des revendications 7, 8, 10 et 12 à 14, caractérisé en ce que les anticorps marqués sont des anticorps modifiés par un élément marqueur choisi parmi les radioéléments, les éléments fluorescents, les éléments luminescents, les enzymes, les chromophores fluorescents, les chromophores absorbant la lumière, la protéine A marquée au PAP (peroxydase - antiperoxydase), l'avidine et la biotine.

16. Procédé selon la revendication 15, caractérisé en ce que l'élément marqueur est la peroxydase de raifort.

17. Procédé de dosage immunologique de la thyroxine $T_4$ ou de la triiodothyronine $T_3$ selon la revendication 1, caractérisé en ce que l'on met en contact l'échantillon contenant la thyroxine $T_4$ (ou la triiodothyronine $T_3$) à doser avec de la thyroglobuline marquée et des anticorps anti-$T_4$ (ou anti-$T_3$) immobilisés sur une phase solide, et on détermine ensuite la quantité de thyroglobuline marquée fixée sur ledit support.

18. Procédé selon la revendication 17, caractérisé en ce que la phase solide contenant les anticorps anti-$T_4$ (ou anti-$T_3$) immobilisés est constituée par des anticorps anti-$T_4$ (ou anti-$T_3$) rendus insolubles par un procédé physicochimique ou immunologique.

19. Procédé selon la revendication 17, caractérisé en ce que la thyroglobuline est marquée par une enzyme.

20. Procédé selon la revendication 17, caractérisé en ce que la thyroglobuline marquée est un composé conjugué thyroglobline anticorps anti-thyroglobuline-enzyme.

21. Procédé de dosage immunologique de la concentration totale en thyroxine $T_4$ et/ou en triiodothyronine $T_3$ d'un échantillon bioloogique, caractérisé en ce qu'il consiste à ajouter à l'échantillon au moins un inhibiteur de la liaison de $T_3$ et/ou $T_4$ avec leurs protéines vectrices pour libérer la $T_3$ et la $T_4$ liées, et à soumettre l'échantillon au procédé de dosage immunologique selon l'une quelconque des revendications 1 à 20.

22. Trousse pour le dosage de la thyroxine $T_4$, par mise en oeuvre du procédé selon l'une quelconque des revendications 1 à 21, caractérisée en ce qu'elle comprend :
    - une série de tubes comportant chacun une phase solide revêtue dans les mêmes conditions de thyroglobuline,
    - une série de flacons contenant des échantillons standards de thyroxine, et
    - un flacon contenant un anti corps anti-$T_4$ marqué.

23. Trousse pour le dosage de la triiodothyronine $T_3$, par mise en oeuvre du procédé selon l'une quelconque des revendications 1 à 21, caractérisée en ce qu'elle comprend :
    - une série de tubes comportant chacun une phase solide revêtue dans les mêmes conditions de thyroglobuline,
    - une série de flacons contenant des échantillons standards de triiodothyronine $T_3$, et
    - un flacon contenant un anticorps anti $T_3$ marqué.

24. Trousse selon la revendication 22, caractérisée en ce que l'anticorps anti-$T_4$ est un composé conjugué d'anticorps anti-$T_4$ monoclonal et d'une enzyme constituée par la peroxydase de raifort.

25. Trousse selon la revendication 24, caractérisée en ce qu'elle comprend de plus :
    - au moins un flacon contenant un chromogène pour la révélation enzymatique,
    - un flacon contenant un tampon substrat pour la révélation enzymatique, et
    - un flacon contenant un acide capable d'arrêter la réaction enzymatique.

26. Trousse selon la revendication 25, caractérisée en ce que le chromogène est le bis-chlorhydrate d'ortho-phénylène-diamine et en ce que l'acide est l'acide oxalique.

## Claims

1. Process for the immunological determination of $T_4$ thyroxine and/or $T_3$ triiodothyronine present in free form in a sample, characterized in that it consists in putting $T_3$ and/or $T_4$ to be determined into competition with thyroglobulin for the sites of anti-$T_3$ and/or anti-$T_4$ antibodies present in limited quantity, and in then determining either the quantity of thyroglobulin fixed to the anti-$T_3$ and/or anti-$T_4$ antibodies, or the quantity of thyroglobulin not fixed to the anti-$T_3$ and/or anti-$T_4$ antibodies.

2. Process according to claim 1, characterized in that the thyroglobulin is immobilized on a solid phase.

3. Process according to claim 2, characterized in that the solid phase containing the immobilized thyroglobulin is formed by thyroglobulin made insoluble by a physicochemical or immunological process.

4. Process for the immunological determination of thyroxine $T_4$ according to claim 2, characterized in that it consists in contacting the $T_4$-containing sample to be determined with the immobilized thyroglobulin and a limited quantity of anti-$T_4$ antibody and in then determining the quantity of anti-$T_4$ antibody fixed to the immobilized thyroglobulin.

5. Process for the immunological determination of $T_3$ triiodothyronine according to claim 2, characterized in that it consists in contacting the $T_3$ triiodothyronine to be determined with the immobilized thyroglobulin and a limited quantity of anti-$T_3$ antibody, and in then determining the quantity of anti-$T_3$ antibody fixed to the immobilized thyroglobulin.

6. Process for the immunological determination of $T_4$ thyroxine and $T_3$ triiodothyronine according to claim 2, characterized in that it consists in contacting the $T_3$- and $T_4$-containing sample with the immobilized thyroglobulin and a limited quantity of anti-$T_3$ antibodies and anti-$T_4$ antibodies, and in then determining the quantity of anti-$T_3$ antibodies fixed to the thyroglobulin and the quantity of anti-$T_4$ antibodies fixed to the thyroglobulin.

7. Process according to claims 4 and 5, characterized in that the anti-$T_3$ and/or the anti-$T_4$ antibodies are labelled antibodies.

8. Process according to claims 4 and 5, characterized in that the anti-$T_3$ or anti-$T_4$ antibodies form a first antibody, and use is made of a second labelled antibody able to be fixed on the first antibody.

9. Process according to claims 4 and 5, characterized in that the anti-$T_3$ or anti-$T_4$ antibodies form a first antibody, use is made of a second antibody able to be fixed on the first antibody, and the first antibody is labelled with a fluorescent chromophore and the second antibody with a light-absorbing chromophore.

10. Process according to claim 6, characterized in that the anti-$T_3$ antibodies and the anti-$T_4$ antibodies are labelled with different atoms and/or molecules.

11. Process according to claim 10, characterized in that the anti-$T_3$ antibodies and the anti-$T_4$ antibodies are labelled with different enzymes.

12. Process according to claim 6, characterized in that the anti-$T_3$ antibodies form a first antibody and the anti-$T_4$ antibodies form a second antibody, and use is made of a third labelled antibody able to be fixed specifically on the first antibody, and a fourth labelled antibody able to be fixed specifically on the second antibody, the third and fourth antibodies being differently labelled.

13. Process for the immunological determination of $T_4$ thyroxine according to claim 1, characterized in that it consists in contacting the thyroxine to be determined with thyroglobulin, a limited quantity of anti-$T_4$ antibodies fixed on a solid phase and labelled anti-$T_3$ antibodies or anti-$T_4$ antibodies, and in then determining the quantity of labelled anti-$T_3$ or anti-$T_4$ antibodies fixed on the solid support via the thyroglobulin.

14. Process for the immunological determination of $T_3$ triiodothyronine according to claim 1, characterized in that it consists in contacting the triiodothyronine to be determined with thyroglobulin, a limited quantity of anti-$T_3$ antibodies fixed on a solid phase, and labelled anti-$T_4$ or anti-$T_3$ antibodies, and in determining the quantity of labelled anti-$T_4$ or anti-$T_3$ antibodies fixed on the solid support via the thyroglobulin.

15. Process according to any one of the claims 7, 8, 10 and 12 to 14, characterized in that the labelled antibodies are antibodies modified by a labelling element selected from radioactive elements, fluorescent elements, luminescent elements, enzymes, fluorescent chromophores, light-absorbing chromophores, PAP (peroxidase-antiperoxidase)-labelled A protein, avidin and biotin.

16. Process according to claim 15, characterized in that the labelling element is horseradish peroxidase.

17. Process for the immunological quantitative determination of $T_4$ thyroxine or $T_3$ triiodothyronine according to claim 1, characterized in that the $T_4$ thyroxine (or $T_3$ triiodothyronine)–containing sample to be determined is contacted with labelled thyroglobulin and anti-$T_4$ (or anti-$T_3$) antibodies immobilized on a solid phase, and then the quantity of labelled thyroglobulin fixed on such support is determined.

18. Process according to claim 17, characterized in that the solid phase containing the immobilized anti-$T_4$ (or anti-$T_3$) antibodies is formed by anti-$T_4$ (or anti-$T_3$) antibodies made insoluble by physicochemical or immunological process.

19. Process according to claim 17, characterized in that the thyroglobulin is labelled with an enzyme.

20. Process according to claim 17, characterized in that the labelled thyroglobulin is a conjugated thyroglobulin antibody/antithyroglobulin-enzyme.

21. Process for the immunological determination of the total $T_4$ thyroxine and/or $T_3$ triiodothyronine concentration of a biological sample, characterized in that it consists in adding to the sample at least one inhibitor of the bonding of $T_3$ and/or $T_4$ with their carrier proteins to release the bonded $T_3$ and $T_4$, and in subjecting the sample to the immunological determination process according to any one of the claims 1 to 20.

22. Kit for the determination of $T_4$ thyroxine, by performing the process according to any one of the claims 1 to 21, characterized in that it comprises
   - a series of tubes each having a solid phase coated in the same conditions with thyroglobulin,
   - a series of flasks containing standard samples of thyroxine, and
   - a flask containing a labelled anti-$T_4$ antibody.

23. Kit for the determination of $T_3$ triiodothyronine by performing the process according to any one of the claims 1 to 21, characterized in that it comprises
   - a series of tubes each having a solid phase coated in the same conditions with thyroglobulin,
   - a series of flasks containing standard samples of $T_3$ triiodothyronine, and
   - a flask containing a labelled anti-$T_4$ antibody.

24. Kit according to claim 22, characterized in that the anti-$T_4$ antibody is a conjugated compound of monochlonal anti-$T_4$ antibody and an enzyme formed by horseradish peroxidase.

25. Kit according to claim 24, characterized in that it also comprises:
   - at least one flask containing a chromogen for enzymatic developing
   - a flask containing a substrate buffer for enzymatic development, and
   - a flask containing an acid able to stop the enzymatic reaction.

26. Kit according to claim 25, characterized in that the chromogen is the bis-chlorohydrate of ortho-phenylene diamine, and the acid is oxalic acid.

**Patentansprüche**

1. Verfahren zur immunologischen Bestimmung von Thyroxin $T_4$ und/oder von Trijodthyronin $T_3$, die in freier Form in einer Probe vorliegen, dadurch gekennzeichnet, daß man das zu bestimmende $T_3$ und/oder $T_4$ mit Thyroglobulin konkurrieren läßt um die Reaktionszentren der Antikörper Anti-$T_3$ und/oder Anti-$T_4$, die in begrenzter Menge vorliegen, und anschließend entweder die Menge des an den Antikörpern Anti-$T_3$ und/oder Anti-$T_4$ fixierten Thyroglobulins oder die Menge des an den Antikörpern Anti-$T_3$ und/oder Anti-$T_4$ nicht-fixierten Thyroglobulins bestimmt.

2. Verfahren nach Anspruch 1, dadurch gekennzeichnet, daß man das Thyroglobulin an einer festen Phase immobilisiert.

3. Verfahren nach Anspruch 2, dadurch gekennzeichnet, daß die das immobilisierte Thyroglobulin enthaltende feste Phase aus Thyroglobulin besteht, das durch ein physikalisch-chemisches Verfahren oder ein immunologisches Verfahren unlöslich gemacht worden ist.

4. Verfahren zur immunologischen Bestimmung von Thyroxin $T_4$ nach Anspruch 2, dadurch gekennzeichnet, daß man die das zu bestimmende $T_4$ enthaltende Probe mit dem immobilisierten Thyroglobulin und einer begrenzten Menge von Antikörpern Anti-$T_4$ in Kontakt bringt und anschließend die Menge der an dem immobilisierten Thyroglobulin fixierten Antikörper Anti-$T_4$ bestimmt.

5. Verfahren zur immunologischen Bestimmung von Trijodthyronin $T_3$ nach Anspruch 2, dadurch gekennzeichnet, daß man die das zu bestimmende Trijodthyronin $T_3$ enthaltende Probe mit dem immobilisierten Thyroglobulin und einer begrenzten Menge von Antikörpern Anti-$T_3$ in Kontakt bringt und anschließend die Menge der an dem immobilisierten Thyroglobulin fixierten Antikörper Anti-$T_3$ bestimmt.

6. Verfahren zur immunologischen Bestimmung von Thyroxin $T_4$ und/oder Trijodthyronin $T_3$ nach Anspruch 2, dadurch gekennzeichnet, daß man die Probe, die das zu bestimmende $T_3$ und $T_4$ enthält, mit dem immobilisierten Thyroglobulin und einer begrenzten Menge von Antikörpern Anti-$T_3$ und Antikörpern Anti-$T_4$ in Kontakt bringt und anschließend die Menge der an dem Thyroglobulin fixierten Antikörper Anti-$T_3$ und die Menge der an dem Thyroglobulin fixierten Antikörper Anti-$T_4$ be-

stimmt.

7. Verfahren nach einem der Ansprüche 4 und 5, dadurch gekennzeichnet, daß es sich bei den Antikörpern Anti-$T_3$ und/oder bei den Antikörpern Anti-$T_4$ um markierte Antikörper handelt.

8. Verfahren nach einem der Ansprüche 4 und 5, dadurch gekennzeichnet, daß die Antikörper Anti-$T_3$ oder Anti-$T_4$ einen ersten Antikörper darstellen und daß man einen zweiten markierten Antikörper verwendet, der an dem ersten Antikörper fixiert werden kann.

9. Verfahren nach einem der Ansprüche 4 und 5, dadurch gekennzeichnet, daß die Antikörper Anti-$T_3$ oder Anti-$T_4$ einen ersten Antikörper darstellen, daß man einen zweiten Antikörper verwendet, der an dem ersten Antikörper fixiert werden kann und daß man den ersten Antikörper durch einen fluoreszierenden Chromophoren markiert und den zweiten Antikörper durch einen Licht absorbierenden Chromophoren markiert.

10. Verfahren nach Anspruch 6, dadurch gekennzeichnet, daß man die Antikörper Anti-$T_3$ und die Antikörper Anti-$T_4$ durch unterschiedliche Atome und/oder Moleküle markiert.

11. Verfahren nach Anspruch 10, dadurch gekennzeichnet, daß man die Antikörper Anti-$T_3$ und die Antikörper Anti-$T_4$ durch zwei unterschiedliche Enzyme markiert.

12. Verfahren nach Anspruch 6, dadurch gekennzeichnet, daß die Antikörper Anti-$T_3$ einen ersten Antikörper darstellen und daß die Antikörper Anti-$T_4$ einen zweiten Antikörper darstellen und daß man einen dritten markierten Antikörper verwendet, der in spezifischer Weise an dem ersten Antikörper fixiert werden kann, und daß man einen vierten markierten Antikörper verwendet, der in spezifischer Weise an dem zweiten Antikörper fixiert werden kann, wobei die dritten und vierten Antikörper auf verschiedene Weise markiert sind.

13. Verfahren zur immunologischen Bestimmung von Thyroxin $T_4$ nach Anspruch 1, dadurch gekennzeichnet, daß man das zu bestimmende Thyroxin mit dem Thyroglobulin, einer begrenzten Menge von Antikörpern Anti-$T_4$, die an einer festen Phase fixiert sind, und von markierten Antikörpern Anti-$T_3$ oder Anti-$T_4$ in Kontakt bringt und anschließend die Menge der markierten Antikörper Anti-$T_3$ oder Anti-$T_4$ bestimmt, die über das Thyroglobulin an dem festen Träger fixiert sind.

14. Verfahren zur immunologischen Bestimmung von Trijodthyronin $T_3$ nach Anspruch 1, dadurch gekennzeichnet, daß man das zu bestimmende Trijodthyronin mit dem Thyroglobulin, einer begrenzten Menge von Antikörpern Anti-$T_3$, die an einer festen Phase fixiert sind, und von markierten Antikörpern Anti-$T_4$ oder Anti-$T_3$ in Kontakt bringt und die Menge der markierten Antikörper Anti-$T_4$ oder Anti-$T_3$, die über das Thyroglobulin an dem festen Träger fixiert sind, bestimmt.

15. Verfahren nach einem der Ansprüche 7, 8, 10 und 12 bis 14, dadurch gekennzeichnet, daß es sich bei den markierten Antikörpern um Antikörper handelt, die durch ein Markierungselement, ausgewählt aus der Gruppe der Radioelemente, der fluoreszierenden Elemente, der lumineszierenden Elemente, der Enzyme, der fluoreszierenden Chromophoren, der Licht absorbierenden Chromophoren, des mit PAP (Peroxydase-Antiperoxydase) markierten Proteins A, Avidin und Biotin, modifiziert worden sind.

16. Verfahren nach Anspruch 15, dadurch gekennzeichnet, daß es sich bei dem Markierungselement um die Meerrettich-Peroxydase handelt.

17. Verfahren zur immunologischen Bestimmung von Thyroxin $T_4$ oder Trijodthyronin $T_3$ naoh Anspruch 1, dadurch gekennzeichnet, daß man die das zu bestimmende Thyroxin $T_4$ (oder Trijodthyronin $T_3$) enthaltende Probe mit dem markierten Thyroglobulin und mit auf einer festen Phase immobilisierten Antikörpern Anti-$T_4$ (oder Anti-$T_3$) in Kontakt bringt und anschließend die Menge des an diesem Träger fixierten markierten Thyroglobulins bestimmt.

18. Verfahren nach Anspruch 17, dadurch gekennzeichnet, daß die feste Phase, welche die immobilisierten Antikörper Anti-$T_4$ (oder Anti-$T_3$) enthält, aus Antikörpern Anti-$T_4$ (oder Anti-$T_3$) besteht, die durch ein physikalisch-chemisches oder immunologisches Verfahren unlöslich gemacht worden sind.

19. Verfahren nach Anspruch 17, dadurch gekennzeichnet, daß das Thyroglobulin durch ein Enzym markiert ist.

20. Verfahren nach Anspruch 17, dadurch gekennzeichnet, daß es sich bei dem markierten Thyroglobulin um eine konjugierte Thyroglobulin-Antikörper-Anti-Thyroglobulin-Enzym-Verbindung handelt.

21. Verfahren zur immunologischen Bestimmung der Gesamtkonzentration an Thyroxin $T_4$ und/oder an Trijodthyronin $T_3$ in einer biologischen Probe, dadurch gekennzeichnet, daß man der Probe mindestens einen Inhibitor für die Bindung von $T_3$ und/oder $T_4$ an ihre Vektor-Proteine zusetzt, um das gebundene $T_3$ und das gebundene $T_4$ freizusetzen, und daß man die Probe dem Verfahren zur immunologischen Bestimmung nach einem der Ansprüche 1 bis 20 unterwirft.

22. Set zur Bestimmung von Thyroxin $T_4$ unter Anwendung des Verfahrens nach einem der Ansprüche 1 bis 21, dadurch gekennzeichnet, daß er umfaßt:
    - eine Reihe von Reagensröhrchen, die jeweils eine feste Phase enthalten, die mit Thyroglobulin unter den gleichen Bedingungen bedeckt worden ist,
    - eine Reihe von Kolben, die Thyroxin-Standardproben enthalten, und
    - einen Kolben, der einen markierten Antikörper Anti-$T_4$ enthält.

23. Set für die Bestimmung von Trijodthyronin $T_3$ durch Anwendung des Verfahrens nach einem der Ansprüche 1 bis 21, dadurch gekennzeichnet, daß er umfaßt:
    - eine Reihe von Reagensröhrchen, die jeweils eine feste Phase enthalten, die mit Thyroglobulin unter den gleichen Bedinungen bedeckt ist,
    - eine Reihe von Kolben, die Trijodthyronin $T_3$-Standardproben enthalten, und
    - einen Kolben, der einen markierten Antikörper Anti-$T_3$ enthält.

24. Set nach Anspruch 22, dadurch gekennzeichnet, daß es sich bei dem Antikörper Anti-$T_4$ um eine konjugierte Verbindung aus einem monoklonalen Antikörper Anti-$T_4$ und einem Enzym, das aus Meerrettich-Peroxydase besteht, handelt.

25. Set nach Anspruch 24, dadurch gekennzeichnet, daß er außerdem umfaßt:
    - mindestens einen Kolben, der ein Chromogen für den Enzym-Nachweis enthält,
    - einen Kolben, der ein Puffersubstrat für den Enzym-Nachweis enthält, und
    - einen Kolben, der eine Säure enthält, welche die enzymatische Reaktion abstoppen kann.

26. Set nach Anspruch 25, dadurch gekennzeichnet, daß es sich bei dem Chromogen um das Bischlorhydrat von ortho-Phenylendiamin handelt und daß es sich bei der Säure um die Oxalsäure handelt.

FIG. 1

FIG. 2

# FIG. 3

# FIG. 4

FIG. 5

FIG. 6